# EUROPÄISCHE PATENTSCHRIFT

(11) **EP 0 898 955 B1**
(45) Veröffentlichungstag und Bekanntmachung des Hinweises auf die Patenterteilung: **14.06.2006**
(21) Anmeldenummer: 98114388.6
(22) Anmeldetag: 31.07.1998
(51) Int. Cl.: C09C 1/00, A61K 8/37, A61Q 17/04, A61K 8/29, A61K 8/40, A61K 8/35, A61K 8/49

(54) **Sonnenschutzmittel mit Ultraspektralschutz**
Sunscreen agent showing ultra-spectral protection
Moyen de protection solaire avec une protection ultra-spectrale

(30) Priorität: 09.08.1997 DE 19734582; 18.10.1997 DE 19746139; 12.11.1997 DE 19750028; 08.07.1998 DE 19830531
(43) Veröffentlichungstag der Anmeldung: 03.03.1999
(73) Patentinhaber: Merck Patent GmbH, 64293 Darmstadt (DE)
(72) Erfinder: Kurz, Thekla, 64285 Darmstadt (DE); Wille, Dorothee, 64291 Darmstadt (DE); Driller, Hansjürgen, 64853 Otzberg (DE); Hitzel, Sabine, 64409 Messel (DE)

(56) Entgegenhaltungen:
- EP-A- 0 782 881
- WO-A-98/01107
- FR-A- 2 646 346
- FR-A- 2 729 132
- US-A- 6 004 567
- R. SAYRE ET AL.: "physical sunscreens" J. SOC. COSMET. CHEM., Bd. 41, Nr. 2, 1990, Seiten 103-109, XP000917589
- DATABASE CHEMICAL ABSTRACTS [Online] STN; access number 119: 34 124, XP002177801 & JP 05 097646 A (POLA KASEI K.K.K.) 20. April 1993 (1993-04-20)
- DATABASE CHEMICAL ABSTRACTS [Online] STN; access number 119: 256 288, XP002177802 & JP 05 156174 A (TEIKOKU KAKO CO., LTD) 22. Juni 1993 (1993-06-22)
- DATABASE CHEMICAL ABSTRACTS [Online] STN; access number 124: 37 394, XP002177803 & JP 07 258031 A (TOPY IND.) 9. Oktober 1995 (1995-10-09)

## Beschreibung

Die vorliegende Erfindung betrifft die Verwendung von Interferenzpigmenten in Kosmetika als Lichtschütz filter.

Während vor etwa 30 Jahren Sonnenlicht aufgrund der Vitamin D-Synthese als heilend und unbedenklich angesehen wurde, hat sich in den letzten Jahren die Einstellung in dieser Beziehung nicht nur aus medizinischer Sicht erheblich geändert. Das Gefahrenpotential, welches sowohl natürliche als auch künstliche Bestrahlung mit Sonnenlicht in sich birgt, ist im Bewußtsein in den Vordergrund gerückt. Insbesondere ist auch eine Verhaltensänderung hervorgerufen worden durch das Wissen über den Einfluß von Sonnenlicht auf die Hautalterung und die Entstehung von Hautkrebs.

Bekanntlich reagiert die Haut empfindlich auf Sonnenstrahlen, welche einen gewöhnlichen Sonnenbrand oder ein Erythem, aber auch mehr oder weniger ausgeprägte Verbrennungen hervorrufen können.

Sonnenstrahlen haben aber auch andere negative Wirkungen: sie bewirken, daß die Haut ihre Elastizität verliert und sich Falten bilden und führen somit zu einer frühzeitigen Alterung. Manchmal kann man auch Dermatosen beobachten, und im extremen Fall kommt es zum Auftreten von Hautkrebs.

Aufgrund dieses Wissens hat sich auch im Sonnenschutz einiges geändert. Während noch vor einigen Jahren das Hauptziel in einem erythemhemmenden UVB-Schutz bestand, wird inzwischen ein Schutz gegen UVA-Strahlung in Sonnenschutzformulierungen mit eingeschlossen. Die UVA-Strahlung ist im wesentlichen Auslöser für die Pigmentierung der Haut.

Es ist auch wünschenswert, Haare gegen photochemische Schäden zu schützen, um Veränderungen von Farbnuancen, ein Entfärben oder Schäden mechanischer Art zu verhindern.

Bekanntlich wird der gefährlichste Teil der Sonnenstrahlen von den ultravioletten Strahlen mit einer Wellenlänge von weniger als 400 nm gebildet. Bekannt ist auch, daß durch das Vorhandensein der Ozonschicht der Erdatmosphäre, die einen Teil der Sonnenstrahlung absorbiert, die untere Grenze der ultravioletten Strahlen, welche die Erdoberfläche erreichen, bei ca. 280 nm liegt.

Daher lag das Hauptziel im Bereich Sonnenschutz bisher eigentlich darin, einen guten Schutz gegen UVB- und UVA-Strahlung zu gewährleisten.

Der Wellenlängenbereich des Sonnenlichts erstreckt sich jedoch nicht nur über den Bereich der UVA- und UVB-Strahlung von 280 bis 400 nm. Der durch das Auge wahrnehmbare Bereich reicht bis zu 800 nm und ist begrenzt durch den Übergang in die langwellige Infrarotstrahlung, welche als Wärmestrahlung wahrgenommen wird. Nach unten geht die Strahlung über in den kurzwelligen UV-Bereich, auch UVC-Strahlung genannt. Dieses ist der Wellenlängenbereich von 100 bis 280 nm.

Sonnenlicht im Wellenlängenbereich von 400 bis 800 nm (VIS-Bereich) und darüber (IR-Bereich) kann in tiefere Hautschichten eindringen und dort in schädigender Weise wirksam werden, so daß gerade auch durch diesen Einfluß des Lichts Hautalterungsprozesse beschleunigt werden können.

Aus dem Stand der Technik sind dabei verschiedene Perlglanzpigment-Zubereitungen bekannt:

In der Publikation Cosmetics & Toiletries 106(3) 1991, S.53-55 werden Perlglanzpigmente, die aufgebaut sind aus Titandioxidschichten auf Glimmer, beschrieben. Die Verwendung dieser Pigmente aufgrund ihres dekorativen Effekts und als UV-Filter in der Kosmetik wird beschrieben. Es werden weiter Sonnenschutzformulierungen angegeben, ein solches Interferenzpigment (Flamenco Ultrafine 130F) und organische UV-Filter enthalten.

Die internationale Patentanmeldungen WO 95/09598 beschreibt Sonnenschutzmittel, die titanisierten Glimmer und organische UV-Filter enthalten. In der internationalen Patentanmeldung WO 98/11107 werden Sonnenschutzzusammensetzungen enthaltend Timiron Super Red (ein Interferenzpigment auf Glimmer-Basis, aufgebaut aus Titandioxid- und Zinnoxid-Schichten) und organische UV-Filter enthalten, beschrieben.

JP-A-07/258031 beschreibt Glimmer-basierte Pigmente, die UV-Strahlung filtern und eine Farbe nahe der Farbe der menschlichen Haut zeigen.

JP-A-05/156174 beschreibt Glimmer-basierte Pigmente, die eine Beschichtung aus Titanoxid-Hydrat bzw. Zinkoxid-Hydrat aufweisen und deren Verwendung in der Kosmetik.

In JP-A-05/97646 werden kosmetische Zusammensetzungen beschrieben, die Pulver enthaltend mindestens 2 Phasen aus Metallcarbiden und Metallnitriden, insbesondere TiC dispergiert in TiN, enthalten. Diese Zusammensetzungen absorbieren IR-Strahlung.

In J. Soc. Cosmet. Chem. 41 (1990) S. 103-109n werden physikalische Lichtschutzmittel, d.h. solche die Licht reflektieren bzw. streuen beschrieben. Dabei werden verschiedene Metalloxide (ZnO, TiO₂, farbige Eisenoxide) gegen Bariumsulfat als Referenz untersucht.

Aufgabe der Erfindung war es daher, kosmetisch gut verträgliche Lichtschutzfilter zur Verfügung zu stellen, welche auch einen Schutz gegen Sonnenlicht im Wellenlängenbereich von 400 bis 800 nm bieten.

Aufgabe der Erfindung war es auch, kosmetische Formulierungen zur Verfügung zu stellen, welche einen Schutz gegen den schädigenden Einfluß von Sonnenlicht sowohl im UV-Bereich als auch im langwelligen Bereich, dem VIS- und IR-Bereich, bieten. Dies bedeutet, daß Formulierungen bereitgestellt werden sollten, die einen Komplettschutz gegen das gesamte, die Erde erreichende Sonnenspektrum, wie es z.B. durch Kleidung geschieht, aufweisen. Die Aufgabe lag also darin, kosmetisch gut verträgliche Formulierungen zur Verfügung zu stellen, welche einen solchen Breitband- oder Ultra-Spektralschutz bieten.

Die Lösung der Aufgabe erfolgt durch Lichtschutzfilter wirkend im Wellenlängenbereich von 400 bis 800 nm (VIS-Bereich) und im Wellenlängenbereich über 800 nm (IR-Bereich), wobei es sich um in kosmetischen Formulierungen lösliche oder unlösliche oder Gemische von löslichen und unlöslichen Substanzen handelt.

Gegenstand der Erfindung ist somit die Verwendung von goldenen, roten, orange-, kupfer- oder körperfarbenen Interferenzpigmenten, wobei die Interferenzpigmente aus plättchenförmigem oder vermahlenen Glimmer mit einem Durchmesser von bis zu 15 µm, welcher beschichtet ist mit SnO₂ und/oder TiO₂, bestehen, oder deren Gemischen in kosmetischen Formulierungen wie Sonnenschutzmitteln, Hautcremes oder -gelen, Haargelen oder kosmetischen Stiften als Lichtschutzfilter im Bereich von 400 bis 800 nm.

Bei diesen Interferenzpigmenten handelt es sich um plättchenförmigen oder vermahlenen Glimmer mit einem Durchmesser von bis zu 15 µm, welcher beschichtet ist mit SnO₂ und/oder TiO₂. durch Eisen oder Cer.

In einer besonderen Ausführungsform dieser Pigmente handelt es sich um Glimmer mit einer dünnen Beschichtung, bestehend aus bis zu einem Gew.-% SnO₂, und einer Beschichtung, bestehend aus 50-70 Gew.-%, bevorzugt 54-60 Gew.-%, TiO₂ mit einer Rutil-Struktur.

Bei den Lichtschutzfltern kann es sich auch um Glimmer mit einer dünnen Beschichtung, bestehend aus bis zu einem Gew.-% SnO₂, und einer Beschichtung, bestehend aus 50-70 Gew.-%, bevorzugt 54-60 Gew.-%, TiO₂ mit einer Anatas-Struktur handeln oder um Glimmer mit einer Beschichtung, bestehend aus 50-70 Gew.-%, bevorzugt 54-60 Gew.-%, TiO₂ mit einer Rutil- oder Anatas-Struktur.

Geeignete Substanzen, die auch als VIS- und/oder als IR-Filter eingesetzt werden können, sind Perlglanzpigmente, bestehend aus Glimmer welche mit Titandioxiden belegt sind, insbesondere handelt es sich hierbei um
- Silberpigmente (Glimmer + TiO₂) mit Partikelgrößen < 200 µm, insbesondere < 15 µm, wie z.B. das im Handel erhältliche Timiron MP 1005® oder MP 1001® oder auch gröbere Fraktionen
- Interferenzpigmente (Glimmer + TiO₂) mit Partikelgrößen < 200 µm, insbesondere mit Partikelgrößen von 5 bis 25 µm, mit goldener, roter, orange-, kupfer- oder körperfarbener Interferenz, wie z.B. Timiron Silk Red® oder Silk Gold® oder Super Red® oder Super Gold® oder Super Copper® oder gröbere Fraktionen oder andere Interferenzfarben und deren Mischungen
- Goldpigmente (Glimmer + TiO₂ und Eisenoxide) mit Partikelgrößen < 200 µm, insbesondere < 5-25 µm oder < 15 µm; ein solches Goldpigment ist z.B. Timiron MP 20® , aber auch gröbere Goldpigmentfraktionen sind geeignet
- Farbpigmente (Glimmer + TiO₂ und Eisenoxide) mit Partielgrößen < 200 µm, insbesondere < 5-25 µm oder < 15 µm; entsprechende Farbpigmente sind z.B. Dichrona® oder Microna® Matte.

Ferner können die VIS-Filter neben ihrer Schutzwirkung im VIS-Bereich auch eine Schutzwirkung im UV- oder IR-Bereich besitzen.

Ein Schutz der Haut vor IR-Strahlung ist ebenfalls sinnvoll und wichtig, da die IR-Strahlung des Sonnenlichts wesentlich zur Erwärmung beiträgt. Diese Wärme wiederum ist bei der UV-bedingten Erythembildung synergistisch, d.h. die Sonnenbrandbildung wird hierdurch gefördert.

Als IR-Schutzfilter kommen hierbei im Prinzip viele für den VIS-Bereich beschriebene Substanzen, insbesondere die Interferenzpigmente, die im längerwelligen Bereich wirken, in Frage. Der Übergang zwischen dem VIS- und dem IR-Bereich ist häufig fließend.

Vorzugsweise werden also im IR-Bereich reflektierende Pigmente eingesetzt. Oftmals wird jedoch das starke "Weißeln" auf der Haut als verbesserungswürdig betrachtet. Die Lösung dieser erfindungsgemäßen Aufgabe wurde durch das Bereitstellen eines neues Inteferenzpigmentes mit Wirkung im IR-Wellenlängenbereich gelöst.

Offenbart ist daher auch ein Interferenzpigment für den IR-Schutz, dadurch gekennzeichnet, daß das Pigment eine weiße Körperfarbe und eine gelbliche, kupfer- oder hautfarbene Interferenzfarbe besitzt.

Dieses Interferenzpigment besteht aus plättchenförmigem oder vermahlenem Glimmer, welcher beschichtet ist mit TiO₂ unterschiedlicher Schichtdicken, und welcher ferner auch mit Eisen oder Cer dotiert sein kann.

Die erfindungsgemäßen Interferenzpigmente besitzen Farbtönungen im Bereich kupferfarben, gelblich und hautfarben-rosa. Zur besseren farblichen Beschreibung können auch die Farbtöne nach Codes aus dem "Pantone Color Formula Guide 1000 "angegeben werden, die dem Fachmann bekannt sind. Folgende Farbtöne sind im besonderen Maße erfindungsgemäß bevorzugt: 726C, 489U, 489C, 712C, 155U, 719U, 1205U oder auch 1205C. Diese Aufzählung ist lediglich als beschreibende, keineswegs als in irgendeiner Weise limitierende Offenbarung aufzufassen. -

Überraschenderweise zeigen die erfindungsgemäßen Pigmente eine weiße Körperfarbe, d.h. die Formulierungen besitzen eine weiße Farbe, aber auf der Haut erscheint dann wie gewünscht eine kupfer- oder haut-rosafarbene Interferenzfarbe. Das unerwünschte "Weißein" tritt hierbei nicht auf.

Die Herstellung der erfindungsgemäßen Interferenzpigmente erfolgt nach den allgemein bekannten Methoden zum fortlaufenden Schicht-Aufbau an Ti(OH)₄ auf Glimmerteilchen (z.B. beschrieben in den Dokumenten US 4,038,099, DE-PS 25 22 572 oder auch EP 0 271 767 B1). Der Prozeß wird dann bei der gewünschten Interferenzfarbe gestoppt.

Die Partikelgröße ist von hoher Wichtigkeit für die Effektivität. Ganz besonders bevorzugt ist eine Partikelgröße von 5 bis 25 µm, da damit eine optimale Schutzwirkung gegen die IR-Strahlung erzielt werden kann.

Wird die Partikelgröße kleiner gewählt als ungefähr 15 µm, dann kann dieses Interferenzpigment auch für den VIS-Bereich hervorragend geeignet sein.

Auch die Interferenzpigmente für den VIS-Bereich können beispielsweise nach den in den genannten Dokumenten beschriebenen Verfahren hergestellt werden.

Erfindungsgemäß können die VIS- und IR-Lichtschutzfilter jeweils für sich allein oder natürlich auch in Kombination, was bevorzugt ist, in kosmetischen Formulierungen wie Sonnenschutzmitteln, Hautcremes, Hautgelen, Haargelen oder kosmetischen Stiften verwendet werden. Hierin sind sie in Kombination sowohl mit anorganischen als auch mit organischen UVA- und UVB-Filtem oder deren Gemischen einsetzbar.

Die erfindungsgemäßen Filter zum Schutz vor VIS- und IR-Strahlung können jeweils in Konzentrationen von 0,5 bis 20 Gew.-%, vorzugsweise 3 bis 10 Gew.-%, in kosmetische Formulierungen eingearbeitet werden. Es ist auf diese Weise möglich Formulierungen herzustellen, in denen bis zu 100 % der eingesetzten Lichtschutzfilter erfindungsgemäße VIS- und/oder IR-Filter sind. Es handelt sich hierbei um Substanzen, die mit Wasser und Ölen in einfacher Weise gelöst, dispergiert oder emulgiert werden.

Die erfindungsgemäßen Lichtschutzfilter lassen sich direkt ohne weitere vorbereitende Maßnahmen in kosmetische Formulierungen einarbeiten.

Diese Substanzen bieten ferner den großen Vorteil, keine toxischen oder allergischen Reaktionen gegenüber der Haut zu zeigen.

Diese kosmetischen Formulierungen weisen einen deutlich verbesserten Schutz gegen den schädlichen Einfluß von Sonnenstrahlen auf.

Da neue Trends dahin gehen, sich gegen UVB-, UVA- sowie auch gegen VIS- oder IR-Strahlung zu schützen, d.h. also einen Komplettschutz gegen das gesamte, die Erde erreichende Sonnenspektrum zu erhalten, wie es z.B. durch Kleidung geschieht, war es auch Aufgabe der Erfindung, kosmetisch gut verträgliche Formulierungen zur Verfügung zu stellen, welche einen solchen Breitband- oder Ultra-Spektralschutz bieten.

Die Lösung der Aufgabe erfolgt durch die Kombination von organischen und/oder anorganischen UV-Filtern, VIS- und IR-Filtern.

Erfindungsgemäß werden also kosmetische Formulierungen bereitgestellt, die Lichtschutzfilter wirkend im Wellenlängenbereich 280 bis 400 nm (UV-Filter), im Wellenlängenbereich 400 bis 800 nm (VIS-Filter) und im längerwelligen Bereich über 800 nm (IR-Filter), enthalten, und somit einen Hyper-Sonnenschutz bieten.

Ebenso ist ein Verfahren zum Schutz der Haut vor Sonnenstrahlen, wobei auf die Haut eine erfindungsgemäße kosmetische Zubereitung aufgetragen wird, Gegenstand der Erfindung.

Die erfindungsgemäßen Formulierungen können sowohl ausschließlich anorganische Lichtschutzfilter als auch Kombinationen von organischen und anorganischen Lichtschutzfiltern enthalten.

Der Gehalt an UV-, VIS- und IR-Filtern in den kosmetischen Formulierungen kann jeweils zwischen 0,5 und 20 Gew.-%, vorzugsweise zwischen 3 und 10 Gew.-%, liegen.

Als VIS- und/oder IR-Filter werden vorzugsweise die in diesem Dokument ausführlich beschriebenen neuen Lichtschutzfilter eingesetzt.

Als UV-Filter kommen anorganische und organische Lichtschutzfilter in Betracht. Als anorganische UV-Filter können dem Fachmann allgemein bekannte UV-Filter wie zum Beispiel solche aus der Gruppe Titandioxid und Zinkoxid eingesetzt werden. Im Handel erhältlich ist beispielsweise auch das Eusolex® T-2000 (Fa. Merck KGaA, Darmstadt), ein mikronisiertes Titandioxid.

Diese anorganischen UV-Filter werden in der Regel in einer Menge von 0,5 bis 20 Gewichtsprozent, vorzugsweise 2 - 10 %, in kosmetische Formulierungen eingearbeitet.

Als geeignete organische UV-Filter kommen alle dem Fachmann bekannten UVA- als auch UVB-Filter in Frage. Für beide UV-Bereiche gibt es viele aus der Fachliteratur bekannte und bewährte Substanzen, beispielsweise seien hier nur Substanzen wie Benzylidencampherderivate (z.B. Eusolex® 6300) oder Phenylbenzimidazol-5-sulfonsäure (Eusolex® 232), Benzoyl- oder Dibenzoylmethane wie Eusolex® 9020 oder Eusolexe® 8020, Benzophenone (Eusolex® 4360), Methoxyzimtsäureester (z.B. Eusolex® 2292), Salicylatderivate (z.B. Eusolex® OS), Octocrylen (Eusolex® OCR), 4-Amino-benzoesäure (PABA), Homosalicylate (HMS) oder auch Octyl Triazone (Uvinul® T 150) aufgeführt.

Diese organischen UV-Filter werden in der Regel in einer Menge von 0,5 bis 10 Gewichtsprozent, vorzugsweise 1-8 Gew.-%, insbesondere bevorzugt 3 bis 6 Gew.-%, in kosmetische Formulierungen eingearbeitet.

Die verschiedenen Lichtschutzfilter lassen sich ohne weiteres in allen möglichen Varianten kombinieren und in kosmetische Formulierungen einarbeiten.

Bevorzugte Ausführungen enthalten beispielsweise folgende Kombinationen: Pigmente zum VIS-Schutz (3-5 Gew.-%), IR-Filter (3-5 Gew.-%) und organische UV-Filter (5-10 Gew.-%); VIS-Filter (3-5 Gew.-%), IR-Filter (3 - 5 Gew.-%) und anorganische und organische UV-Filter (5-20 Gew.-%).

Es handelt sich um Substanzen, die sich mit Wasser und Ölen in einfacher Weise lösen, dispergieren oder emulgieren lassen. Sie verteilen sich gleichmäßig in den herkömmlichen kosmetischen Trägern und können insbesondere in Fetträgern einen kontinuierlichen Film bilden. Sie können auf diese Weise auf die Haut aufgetragen werden, um einen wirksamen Schutzfilm zu bilden.

Die Formulierungen besitzen einen Totalschutz gegen Sonneneinstrahlung und auch eine langanhaltende Schutzwirkung.

Die VIS- und IR-Filter weisen eine hohe chemische Stabilität, d.h. keine Hydrolysierbarkeit, keine Photooxidierbarkeit, keine Oxidierbarkeit, hohe Thermostabilität und hohe Schweißfestigkeit auf.

Mit Hilfe der erfindungsgemäßen VIS- und IR-Filter können kosmetische Zubereitungen hergestellt werden, die in einem deutlich breiteren Wellenlängenbereich schützend wirken und Strahlung absorbieren.

Gegebenenfalls können die erfindungsgemäßen Sonnenschutzmittel auch eine oder mehrere chemische Substanzen mit selbstbräunenden Eigenschaften enthalten.

Als chemische Substanzen mit selbstbräunenden Eigenschaften können alle dem Fachmann bekannten natürlichen und synthetischen Substanzen, welche zur Herstellung von kosmetischen Formulierungen geeignet sind, eingesetzt werden. Solche können sowohl pflanzliche Extrakte als auch synthetische Selbstbräuner, wie z.B. Dihydroxyaceton oder α-Ketole, sein.

Die erfindungsgemäßen Formulierungen besitzen einen Totalschutz gegen Sonneneinstrahlung und auch eine langanhaltende Schutzwirkung.

Gegenstand der Erfindung ist der Lichtschutz im sichtbaren Wellenlängenbereich von 400 - 800 nm (VIS-Schutz).

Die VIS-Filter können neben ihrer Schutzwirkung im VIS-Bereich auch eine Schutzwirkung im UV-oder IR-Bereich besitzen.

Ferner können die erfindungsgemäßen Interferenzpigmente mit weißer Körperfarbe für den IR-Schutz auch eine Schutzwirkung im VIS-Bereich besitzen.

Weiterhin können die erfindungsgemäßen Formulierungen auch zur vorbeugenden Behandlung von Entzündungen und Allergien der Haut sowie auch in bestimmten Fällen zur Verhütung bestimmter Krebsarten verwendet werden.

Die erfindungsgemäße Zubereitung wird als Mittel zum Schutz der menschlichen Epidermis oder der Haare oder auch der sensibilisierten Haare oder als Sonnenschutzmittel verwendet.

Mit "sensibilisierten Haaren" sind Haare gemeint, welche einer Dauerwellenbehandlung, einem Färbe- oder Entfärbeprozeß unterzogen worden sind.

Ebenso ist ein Verfahren zum Schutz der Haare vor Sonneneinstrahlung, wobei auf die Haare eine erfindungsgemäße kosmetische Zubereitung aufgetragen wird, Gegenstand der Erfindung.

Die erfindungsgemäße kosmetische Zubereitung wird zum Schutz menschlicher Epidermis gegen Sonneneinstrahlung verwendet. Dabei liegt sie in verschiedenen, für diesen Typ üblicherweise verwendeten Formen vor. So kann sie insbesondere als Lotion oder Emulsion, wie als Creme oder Milch (O/W, W/O), oder in Form öliger oder ölig-alkoholischer Lotionen, Emulsionen, wie als Creme oder als Milch, in Form ölig-alkoholischer, ölig-wäßriger oder wäßrig-alkoholischer Gele oder als feste Stifte vorliegen oder als Aerosol konfektioniert sein.

Die Formulierung kann kosmetische Adjuvanzien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfüms, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugte Ausführungsform der Erfindung ist eine Emulsion, welche als Schutzcreme oder -milch vorliegt und einerseits außer einem oder mehreren Lichtschutzfiltern mindestens einen VIS- und/oder IR-Filter, und andererseits bei einer Zubereitung für den Ultraspektralschutz außer der erfindungsgemäßen Kombination von Lichtschutzfiltern, Fettalkohole, Fettsäuren, Fettsäureester, insbesondere Triglyceride von Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf Basis von natürlichen oder synthetischen Ölen und Wachsen, Lanolin, Fettsäureestem, insbesondere Triglyceriden von Fettsäuren, oder ölig-alkoholische Lotionen auf Basis eines Niedrigalkohols, wie Ethanol, oder eines Glycols, wie Propylenglykol, und/oder eines Polyols, wie Glycerin, und Ölen, Wachsen und Fettsäureestem, wie Triglyceriden von Fettsäuren, dar.

Die erfindungsgemäße kosmetische Zubereitung kann auch als alkoholisches Gel vorliegen, welches einen oder mehrere Niedrigalkohole oder -polyole, wie Ethanol, Propylenglycol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpem.

Gegenstand der Erfindung sind auch kosmetische Sonnenschutzmittel, die mindestens die erfindungsgemäße Kombination von UV-, VIS- und IR-Filter zur Erzielung eines Hyper-Sonnenschutzes enthalten.

Gegenstand der Erfindung sind auch kosmetische Sonnenschutzmittel, die mindestens einen oder mehrere der beschriebenen VIS-Lichtschutzflter enthalten. Gegenstand der Erfindung sind auch kosmetische Sonnenschutzmittel, die mindestens einen der beschriebenen IR-Filter bzw. mindestens eines der erfindungsgemäßen Interferenzpigmente mit weißer Körperfarbe für den IR-Schutz enthalten.

Ist eine Zubereitung als Aerosol konfektioniert, verwendet man in der Regel die üblichen Treibmittel, wie Alkane, Fluoralkane und Chlorfluoralkane.

Soll das erfindungsgemäße Mittel natürliche oder sensibilisierte Haare vor Sonneneinstrahlung schützen, so kann es als Shampoo, Lotion, Gel oder Emulsion zum Ausspülen vorliegen, wobei die jeweilige Formulierung vor oder nach dem Schampoonieren, vor oder nach dem Färben oder Entfärben, vor oder nach der Dauerwelle aufgetragen wird; oder das Mittel liegt als Lotion oder Gel zum Frisieren und Behandeln, als Lotion oder Gel zum Bürsten oder Legen einer Wasserwelle, als Haarlack, Dauerwellmittel, Färbe- oder Entfärbemittel der Haare vor. Dieses Mittel kann außer den erfindungsgemäßen Lichtschutzfiltern (VIS- und/oder IR-Filter) oder der erfindungsgemäßen Kombination von Lichtschutzfiltern verschiedene, in diesem Mitteltyp verwendete Adjuvanzien enthalten, wie grenzflächenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die erfindungsgemäßen kosmetischen Zubereitungen können mit Hilfe von Techniken hergestellt werden, die dem Fachmann wohl bekannt sind.

Die folgenden Beispiele sollen die Erfindung näher erläutem. Alle %-Angaben sind Gewichtsprozent.

### Beispiel 1

Aus folgenden Komponenten stellt man ein erfindungsgemäßes Sonnenschutzmittel enthaltend VIS-Lichtschutzfilter her.

| | | Gew.- % |
|---|---|---|
| A Eusolex® 9020 (Art.-Nr. 105844) | (1) | 1.00 |
| Eusolex® OCR (Art.-Nr. 105377) | (1) | 3.00 |
| Arlatone 983 S | (2) | 1.50 |
| Arlatone 985 | (2) | 2.20 |
| Brij 76 | (2) | 1.50 |
| Miglyol 812 Neutralöl | (3) | 9.50 |
| B Eusolex® VIS | (1) | 5.00 |
| Sorbitol F flüssig (Art.-Nr. 102993) | (1) | 2.50 |
| Propandiol-1,2 (Art.-Nr. 107478) | (1) | 2.50 |
| Konservierungsmittel | | q.s. |
| demin. Wasser | | ad 100.00 |
| C Carbomer 934 | (4) | 0.50 |
| D Tris(hydroxymethyl)-aminomethan | (1) | 0.36 |
| (Art.-Nr. 108386) | | |
| demin. Wasser | | 9.64 |

Zur Herstellung der Phase B werden alle Komponenten intensiv miteinander vermischt. Anschließend wird C hinzugegeben und durch Rühren untergemischt. Die so hergestellte Mischung läßt man ruhen und quellen bis eine homogene Mischung entstanden ist. Inzwischen wird zur Herstellung der Phase D Tris(hydroxymethyl)-aminomethan mit der angegebenen Menge Wasser vermischt. D wird zu der zuvor hergestellten Mischung B-C hinzugefügt und so lange gerührt, bis eine homogene Phase entstanden ist. Die so erhaltene Phase B-D wird auf 80° C erwärmt. Gleichzeitig werden alle Komponenten der Phase A miteinander vermischt und auf 75° C erwärmt. Mit der erwärmten Phase A wird langsam unter Rühren die erwärmte Phase B-D vermischt. Anschließend läßt man abkühlen.

Als Konservierungsmittel können
0.05 % Propyl-4-hydroxybenzoat (Art.-Nr. 107427) oder
0.15 % Methyl-4-hydroxybenzoat (Art.-Nr. 106757)
verwendet werden.

### Bezugsquellen:

(1) Merck KGaA, Darmstadt
(2) ICI, Essen
(3) HülsTroisdorf AG, Witten
(4) Goodrich, Neuss

### Beispiel 2

Aus folgenden Komponenten stellt man eine erfindungsgemäße Sonnenschutzcreme (O/W) mit Ultra-Speictralschutz enthaltend anorganische Lichtschutzfilter her.

| | | Gew. % |
|---|---|---|
| A Arlatone 983 S | (2) | 1.50 |
| Arlatone 985 | (2) | 2.20 |
| Brij 76 | (2) | 1.50 |
| Miglyol 812 Neutralöl | (3) | 9.50 |
| B Eusolex® T-2000 (Art.-Nr. 105373) | (1) | 5.00 |
| Eusolex® VIS | (1) | 5.00 |
| Timiron Silk Gold® (Art.-Nr. 117239) | (1) | 2.50 |
| Timiron Silk Red® (Art.-Nr. 117240) | (1) | 2.50 |
| Sorbitol F flüssig (Art.-Nr. 102993) | (1) | 2.50 |
| Propandiol-1,2 (Art.-Nr. 107478) | (1) | 2.50 |
| Konservierungsmittel | | q.s. |
| demin. Wasser | | ad 100.00 |
| C Carbomer 934 | (4) | 0.50 |
| D Tris(hydroxymethyl)-aminomethan | (1) | 0.36 |
| (Art.-Nr. 108386) | | |
| demin. Wasser | | 9.64 |

Als Konservierungsmittel können
0.05 % Propyl-4-hydroxybenzoat (Art.-Nr. 107427) oder
0.15 % Methyl-4-hydroxybenzoat (Art.-Nr. 106757)
verwendet werden.

### Herstellung:

Zu der vereinigten Phase B gibt man das Carbomer 934 und läßt die Mischung bis zur Homogenität quellen. Dann gibt man die vorgelöste Phase D hinzu und rührt, bis die Phasen homogen gemischt sind. Dieses Gemisch wird nun auf 80 °C erwärmt, ebenso wird die Phase A gemischt und auf 75 °C erwärmt. Anschließend wird das Gemisch B-D unter Rühren in die Phase A eingebracht und bis zur Abkühlung sorgfältig gerührt.

### Bezugsquellen:

(1) Merck KGaA, Darmstadt
(2) ICI, Essen
(3) HülsTroisdorf AG, Witten
(4) Goodrich, Neuss

### Beispiel 3

Aus folgenden Komponenten stellt man eine erfindungsgemäße Sonnenschutzcreme (O/W) mit Ultra-Spektralschutz enthaltend anorganische und organische Lichtschutzfilter her.

| | | Gew. % |
|---|---|---|
| A Eusolex® 9020 (Art.-Nr. 105844) | (1) | 1.00 |
| Eusolex® 2292 (Art.-Nr. 105382) | (1) | 3.00 |
| Eusolex® 6300 (Art.-Nr. 5385) | (1) | 1.00 |
| Arlatone 983 S | (2) | 1.50 |
| Arlatone 985 | (2) | 2.20 |
| Brij 76 | (2) | 1.50 |
| Miglyol 812 Neutralöl | (3) | 9.50 |
| B Eusolex® VIS | (1) | 5.00 |
| Timiron Silk Gold® (Art.-Nr. 117239) | (1) | 2.50 |
| Timiron Silk Red® (Art.-Nr. 117240) | (1) | 2.50 |
| Sorbitol F flüssig (Art.-Nr. 102993) | (1) | 2.50 |
| Propandiol-1,2 (Art.-Nr. 107478) | (1) | 2.50 |
| Konservierungsmittel | | q.s. |
| demin. Wasser | | ad 100.00 |
| C Carbomer 934 | (4) | 0.50 |
| D Tris(hydroxymethyl)-aminomethan | (1) | 0.36 |
| (Art.-Nr. 108386) | | |
| demin. Wasser | | 9.64 |

Als Konservierungsmittel können
0.05 % Propyl-4-hydroxybenzoat (Art.-Nr. 107427) oder
0.15 % Methyl-4-hydroxybenzoat (Art.-Nr. 106757)
verwendet werden.

### Herstellung:

Zu der vereinigten Phase B gibt man das Carbomer 934 und läßt die Mischung bis zur Homogenität quellen. Dann gibt man die vorgelöste Phase D hinzu und rührt, bis die Phasen homogen gemischt sind. Dieses Gemisch wird nun auf 80 °C erwärmt, ebenso wird die Phase A gemischt und auf 75 °C erwärmt. Anschließend wird das Gemisch B-D unter Rühren in die Phase A eingebracht und bis zur Abkühlung sorgfältig gerührt.

### Bezugsquellen:

(1) Merck KGaA, Darmstadt
(2) ICI, Essen
(3) HülsTroisdorf AG, Witten
(4) Goodrich, Neuss

## Patentansprüche

1. Verwendung von goldenen, roten, orange-, kupfer- oder körperfarbenen Interferenzpigmenten, wobei die Interferenzpigmente aus plättchenförmigem oder vermahlenen Glimmer mit einem Durchmesser von bis zu 15 µm, welcher beschichtet ist mit SnO₂ und/oder TiO₂, bestehen, oder deren Gemischen in kosmetischen Formulierungen wie Sonnenschutzmitteln, Hautcremes oder-gelen, Haargelen oder kosmetischen Stiften als Lichtschutzfilter im Bereich von 400 bis 800 nm.

2. Verwendung gemäß Anspruch 1, **dadurch gekennzeichnet, dass** es sich bei den Interferenzpigmenten um Glimmer mit einer dünnen Beschichtung, bestehend aus bis zu einem Gew.-% SnO₂, und einer Beschichtung, bestehend aus 50-70 Gew.-%, bevorzugt 54-60 Gew.-%, TiO₂ mit einer Rutil-Struktur handelt.

3. Verwendung gemäß Anspruch 1, **dadurch gekennzeichnet, dass** es sich bei den Interferenzpigmenten um Glimmer mit einer dünnen Beschichtung, bestehend aus bis zu einem Gew.-% SnO₂, und einer Beschichtung, bestehend aus 50-70 Gew.-%, bevorzugt 54-60 Gew.-%, TiO₂ mit einer Anatas-Struktur handelt.

4. Verwendung gemäß Anspruch 1, **dadurch gekennzeichnet, dass** es sich bei den Interferenzpigmenten um Glimmer mit einer Beschichtung, bestehend aus 50-70 Gew.-%, bevorzugt 54-60 Gew.-%, TiO₂ mit einer Rutil- oder Anatas-Struktur handelt.

5. Verwendung gemäß mindestens einem der vorstehenden Ansprüche, **dadurch gekennzeichnet, dass** das Interferenzpigment eine weiße Körperfarbe und eine gelbliche, kupfer- oder hautfarbene Interferenzfarbe besitzt.

6. Verwendung gemäß mindestens einem der vorstehenden Ansprüche, **dadurch gekennzeichnet, dass** das Interferenzpigment eine Partikelgröße von 5 bis 25 µm besitzt.

7. Verwendung gemäß mindestens einem der vorstehenden Ansprüche, **dadurch gekennzeichnet, dass** die kosmetische Formulierung anorganische und/oder organische UVA- und UVB-Filter enthält.

## Claims

1. Use of golden, red, orange-, copper- or body- coloured interference pigments, the interference pigments consisting of platelet-shaped or ground mica having a diameter of up to 15 µm, which is coated with SnO₂ and/or TiO₂ or mixtures thereof in cosmetic formulations such as sunscreens, skin creams, skin gels, hair gels or cosmetic sticks as light protection filters in the range from 400 to 800nm.

2. Use according to Claim 1, **characterized in that** the interference pigments are mica having a thin coating consisting of up to 1% by weight of SnO₂, and a coating consisting of 50-70% by weight, preferably 54-60% by weight, of TiO₂ having a rutile structure.

3. Use according to Claim 1, **characterized in that** the interference pigments are mica having a thin coating consisting of up to 1% by weight of SnO₂, and a coating consisting of 50-70% by weight, preferably 54-60% by weight, of TiO₂ having an anatase structure.

4. Use according to Claim 1, **characterized in that** the interference pigments are mica having a coating consisting of 50-70% by weight, preferably 54-60% by weight, of TiO₂ having a rutile or anatase structure.

5. Use according to at least one of the preceding claims, **characterized in that** the interference pigment has a white body colour and a yellowish, copper- or skin-coloured interference colour.

6. Use according to at least one of the preceding claims, **characterized in that** the interference pigment has a particle size of 5-25 µm.

7. Use according to at least one of the preceding claims **characterized in that** the cosmetic formulation comprises inorganic and/or organic UVA and UVB filters.

## Revendications

1. Utilisation de pigments d'interférence de couleur dorée, rouge, orange, cuivrée ou de leur propre couleur, les pigments d'interférence étant constitués de mica en forme de paillettes ou broyé, ayant un diamètre allant jusqu'à 15 µm, qui est revêtu avec SnO₂ et/ou TiO₂, ou de leurs mélanges dans des formulations cosmétiques telles que des agents de protection solaire, des crèmes ou des gels pour la peau, des gels capillaires ou des crayons cosmétiques, comme filtres de protection contre la lumière dans la gamme de 400 à 800 nm.

2. Utilisation selon la revendication 1, **caractérisée en ce qu'**il s'agit pour les pigments d'interférence de mica avec un revêtement mince, constitué de jusqu'à un % en poids de SnO₂, et d'un revêtement, constitué de 50-70 % en poids, de préférence de 54-60 % en poids, de TiO₂ avec une structure rutile.

3. Utilisation selon la revendication 1, **caractérisée en ce qu'**il s'agit pour les pigments d'interférence de mica avec un revêtement mince, constitué de jusqu'à un % en poids de SnO₂ et d'un revêtement, constitué de 50-70 % en poids, de préférence de 54-60 % en poids, de TiO₂ avec une structure anatase.

4. Utilisation selon la revendication 1, **caractérisée en ce qu'**il s'agit pour les pigments d'interférence de mica avec un revêtement, constitué de 50-70 % en poids, de préférence de 54-60 % en poids, de TiO₂ avec une structure rutile ou anatase.

5. Utilisation selon au moins une des revendications précédentes, **caractérisée en ce que** le pigment d'interférence possède une couleur propre blanche et une couleur d'interférence jaunâtre, cuivrée ou de couleur chair.

6. Utilisation selon au moins une des revendications précédentes, **caractérisée en ce que** le pigment d'interférence possède une taille de particules de 5 à 25 *µ*m.

7. Utilisation selon au moins une des revendications précédentes, **caractérisée en ce que** la formulation cosmétique contient un filtre UVA et UVB inorganique et/ou organique.
